Europäisches Patentamt

(19) European Patent Office

Office européen des brevets

(11) Publication number: **0 011 322**
**B1**

(12) **EUROPEAN PATENT SPECIFICATION**

(45) Date of publication of patent specification: **05.05.82**

(51) Int. Cl.³: **A 61 K 31/72, C 08 B 37/00**

(21) Application number: **79200596.9**

(22) Date of filing: **17.10.79**

(54) Anticoagulant agent, process for the preparation thereof and pharmaceutical preparations containing same.

(30) Priority: **01.11.78 SE 7811306**

(43) Date of publication of application:
**28.05.80 Bulletin 80/11**

(45) Publication of the grant of the patent:
**05.05.82 Bulletin 82/18**

(84) Designated Contracting States:
**AT BE CH DE FR GB IT NL SE**

(56) References cited:
**GB - A - 740 152**
**US - A - 2 832 766**

**THE JOURNAL OF BIOLOGICAL CHEMISTRY, vol. 252, no. 7, April 10, 1977,**
**C.M.F. CASSARO: "Distribution of sulfated mucopolysaccharides in invertebrates", pages 2254—2261**

**JOURNAL OF AMERICAN CHEMICAL SOCIETY, vol. 78, 1956,**
**S.L. BURSON et al.: "Isolation and purification of mactins, heparinlike anticoagulants from mollusca" pages 5874—5878**

(73) Proprietor: **Akzo N.V.**
**Velperweg 76**
**NL-6824 BM Arnhem (NL)**

(72) Inventor: **Rothman, Gunnvör**
**2.V. Mellanvagen**
**Hölviksnäs (SE)**
Inventor: **Rothman, Ulf Sven Erik**
**2.V. Mellanvagen**
**Hölviksnäs (SE)**

(74) Representative: **van 't Holt, Hendrik et al,**
**Postbus 20**
**NL-5340 BH Oss (NL)**

Courier Press, Leamington Spa, England.

Anticoagulant agent, process for the preparation thereof and pharmaceutical preparations containing same

The present invention deals with anticoagulants, i.e. drugs that inhibit blood coagulation, and more specifically, with a new anticoagulant, with a method of preparing it and with pharmaceutical preparations containing same.

Blood coagulation is controlled by a complicated mechanism which has only been partly investigated even today. According to the cascade theory, coagulation occurs through the formation of insoluble fibrin under the interaction of a number of various factors. According to this theory, the final step in the coagulation process implies that a factor X is activated to factor $X_A$, whereupon this activated factor $X_A$ enables blood prothrombin to be converted into the enzyme thrombin which, in turn, catalyzes the conversion of fibrinogen to fibrin. Normally, blood coagulation is counteracted by certain substances interfering in the coagulation process, whereby coagulation is inhibited. More specifically, coagulation is counteracted by a protein called antithrombin III (AT III) which slowly neutralizes and inhibits thrombin and factor $X_A$. Normally, the blood concentration of AT III is enough to inhibit coagulation and to maintain blood in a sufficiently fluid state.

It has been established that the action of AT III is highly accelerated by so-called heparin, which is a sulfated mucopolysaccharide. Heparin was first discovered in liver substance by the American W.H. Howell in 1916. Later, concentrations of heparin were also found in the lungs and gut. Pure heparin was first prepared by E. Jorpes. Heparin acts by building a complex with AT III, and this complex is highly effective in inhibiting above all the activation of factor $X_A$ but also, though to a much lesser extent, the enzyme thrombin. It is to be noted that heparin alone exerts no anticoagulant action. Combination with AT III, the heparin cofactor, is necessary.

It is in many circumstances desirable to be able to prevent normal coagulation, for example in vivo in operative surgery as well as in post-operative treatment to prevent thrombosis (formation of emboli). It is even desirable to prevent coagulation in vitro, for instance in blood analysis tests in the laboratory. This can be achieved by the addition of heparin, which together with cofactor AT III, effectively inhibits clotting. Particularly in blood analysis tests in the laboratory, clotting is inhibited in a practical manner by coating the inside of test tubes, in which blood is collected, with heparin. Prophylactic treatment for the prevention of post-operative thrombosis is achieved by the administration of low doses of heparin. It has been shown, however, that treatment is often not as effective as expected. This is due to 2 reasons. Firstly, heparin is, as already mentioned, dependent on a cofactor (AT III) for its activity. And even if heparin is given in a dosage which per se may be adequate in inhibiting thrombosis, treatment is ineffective unless cofactor AT III is present in a sufficient quantity. In this concentration it has been shown that AT III concentrations below normal levels occur in a number of cases, e.g. in patients suffering from hepatic damage, cancer or gramnegative sepsis as well as from disseminated intravasal coagulation, a serious condition following major trauma, which bring about an expenditure of the body's coagulation factors. Secondly, it is known, moreover, that heparin induces a reduction in the activity of the heparin-AT III complex, i.e. increasing the amount of heparin given does not bring about a corresponding increase in coagulation inhibition. Now, both heparin and the heparin-AT III complex are rapidly eliminated by the reticuloendothelial system of the body. It is thus clear that anticoagulant treatment with heparin has limitations. An important limitation is above all, the requirement for a cofactor being available and that it is available in a certain concentration, in order that treatment shall have the desired effect. Admittedly, it has been suggested to prepare pure cofactor AT III in order to administer it to those patients having reduced concentrations of that factor, thus restoring a desired plasma concentration, prior to treatment with heparin. However, that means that treatment is being complicated as compared with treatment with only one anticoagulant.

It would appear from the above that there is a definite need for a good supply of anticoagulants. Moreover, it would be a great advantage having an anticoagulant which acts without cofactor.

The sources used at present for the extraction of heparin are the liver, gut and lungs, and porcine gut is preferably used. Owing to the ever growing used of heparin in, for instance, blood transfusions and post-operative treatment with so-called low-dose heparin for the prevention of thromboembolic complications, there is a risk of shortage, taking into account the fact that raw material source for heparin do not cover the demand. In view of this more or less successful attempts have been made to prepare semisynthetic heparins. Moreover, heparin raw material sources other than the ones used hitherto have been looked for, and it has thereby been found that mollusca, e.g. mussels, contain heparin-like substances. There is thus a description in the paper by L.J. Thomas "A heparin-like blood anticoagulant from surf claims", Univ. Microfilms (Ann Arbor, Mich.), Publ. No. 4951; Dissertation Abstr. 13, 173 (1953) of the presence of a heparin-like anticoagulant drug in mussels. See also Biol.

Bulletin *106* (1954), p. 129. Moreover, a description of the presence and preparation of heparin-like substances called mactins (Mactin-A and Mactin-B) from mussels is to be found in Frommhagen, L.H. et al, "Heparin-like anticoagulants from mollusca", Proc. Soc. Exptl. Biol. Med., 82 (1953) p. 280—283 as well as in Burson, S.L. et al., "Isolation and Purification of Mactins, Heparin-like Anticoagulants from Mollusca", J. Am. Chem. Soc., 78 (1956), p. 5874—5878.

In this connection, it is also known that leeches contain an anticoagulant agent, hirudin, which does not require the presence of a cofactor for its effect. Hirudin is a protein substance with a molecular weight of approximately 16.000 and it was used in the beginning of this century in the treatment of thrombotic disease. However, this treatment was abandoned because hirudin, in view of its character of foreign albumin substance, builds antibodies entailing risk for anaphylactic shock for the patient. Furthermore, the supply of hirudin is minimal.

According to the present invention, a new anticoagulant agent is produced which, just like heparin, inhibits coagulation yet is different from heparin and other heparin-like substances known hitherto owing to its acting through a different mechanism. As mentioned above, the anticoagulant drugs known hitherto act partly through combination with a cofactor and partly through an anticoagulant action dependent mostly on inhibition of the formation of factor $X_A$. In contrast thereof, the anticoagulant agent according to the invention does not require the co-operation of a cofactor and, furthermore, the anticoagulant action is due to inhibition of thrombin only, i.e. an inhibition of factor $X_A$ does not take place.

The anticoagulant agent according to the invention is characterized by the fact that it is a sulphated polysaccharide prepared by sulphation of a polysaccharide obtained from mollusca, or a pharmaceutically acceptable salt thereof, said sulphated polysaccharide having selective thrombin inhibiting action against the formation of fibrin from fibrinogen in the absence of a cofactor.

The term "polysaccharide" as used herein means polysaccharides in a broad sense, i.e. including also mucopolysaccharides.

The mollusca raw material source is, preferably, mussels and specially common sea mussels (Mytilus). A raw material source particularly preferred is Mytilus edulis.

The preparation method of the new anticoagulant agent is characterized by the fact that the initial material, which is made up of flesh from mollusca, is processed for the purpose of extraction of the polysaccharides found therein and that the polysaccharides are then sulphated and eventually, if desired, converted into a pharmaceutically acceptable salt.

As mentioned above, the initial material is preferably made up of mussels, specially common sea mussels (Mytilus) and Mytilus edulis is particularly preferred. It is preferred according to the invention to effectuate the extraction by boiling the initial material in a water solution under a pressure of at least 1 atm and a temperature of at least 100°C, whereby the polysaccharide is obtained in the decoction. Sulphation is preferably carried out by processing the polysaccharide obtained with chlorsulphonic acid in the presence of pyridine. The sulphated polysaccharide is extracted from the reaction solution and then purified in the usual manner.

Hereunder is a detailed description of the invention and its advantages.

On examination of the mollusca extract obtained by boiling mollusca flesh in water solution, it was found that the extract contained polysaccharide. Normally, this polysaccharide is not sulphated as evidenced by the fact that it does not induce metachromasia together with toluidine blue (colour change from blue to violet) nor does it exhibit anticoagulant properties. In a few unreproduceable cases the polysaccharide contained in the extract has produced metachromasia together with toluidine blue and has exhibited an anticoagulant action. Irrespective of whether the extracted polysaccharide exhibits anticoagulant properties or not it is transformed into the anticoagulant agent of the invention by the sulphation process described in the invention. As mentioned before, this anticoagulant agent is not identical with heparin. It differs from the latter through the fact that it acts without cofactor and selectively exhibits thrombin without inhibiting factor $X_A$.

The fact that the anticoagulant agent described in the invention acts without cofactor can easily be shown by two thrombin-neutralizing tests. In the first test a reagent solution containing thrombin, fibrinogen and cofactor AT III is used whereas in the second test a reagent solution containing thrombin and fibrinogen, but no cofactor, is used. Each one of the reagent solutions is tested regarding heparin and the anticoagulant agent of the present invention, and it is found that heparin exhibits anticoagulant properties only in the presence of the reagent solution containing the cofactor, whereas the anticoagulant agent described in the invention exhibits anticoagulant properties in the presence of both reagent solutions. This result shows that the anticoagulant agent described in the invention inhibits thrombin and that it thereby acts without cofactor. The fact that the anticoagulant agent described in the invention does not inhibit factor $X_A$ is shown by adding the new anticoagulant agent to a given quantity of factor $X_A$. A reagent is then added which, under the influence of factor $X_A$, can split a colour giving group to be spectrographically gauged at a given wave length. Lack of absorption at the actual wave length indicates complete inhibition of factor $X_A$ whereas, conversely, high absorption indicates lack of inhibition of factor $X_A$. Experiment with this test clearly indicates that the anticoagulant agent described in the invention does not inhibit factor $X_A$.

Furthermore, it is to be noted that the initial material is of importance in order to obtain the required end-product. Thus, the anticoagulant agent described in the invention cannot be obtained by desulphation and renewed sulphation of common heparin.

It has been found that a suitable initial material for the preparation of the anticoagulant agent described in the invention is mollusca (mussel, octopus, snail), preferably such mollusca as mussels and oysters. Common sea mussels are preferred (Mytilus) and particularly Mytilus edulis. Canned mussel is prepared in several places, whereby great quantities of decoction waste are are present destroyed. However, this decoction waste constitutes an excellent raw material source for the present invention, in view of the fact that it contains the required polysaccharide in extractable form. In order to prepare the anticoagulant described in the invention it is thus enough to extract the polysaccharide from the decoction waste, sulphate it and then cleanse the product from impurities. It may be mentioned as an example that on boiling 450 kg mussel at a pressure of 5 atm, (506.25 kPa), 135 liters of decoction is obtained as waste, from which broth 0,2 kg of the anticoagulant described in the invention is obtained after extraction and sulphation.

Regarding the extraction process from the initial mollusca material, it may generally be said that it occurs through extraction with water solution at a pressure of 1 atm (101.325 kPa) (atmospheric pressure) at least, preferably about 2—15 atm (202.65 to 1519.875 kPa) and especially about 4—6 atm, (405.3—607.95 kPa) and at a temperature of 100°C at least, preferably at a temperature corresponding to saturated steam temperature at chosen pressure. The process of extraction should last long enough to release to a great extent the required polysaccharide. The release of essentially all the required polysaccharide should be aimed at. For that purpose, the time allowed for the extraction process should not fall short of approximately 2 minutes, is suitably about 2—15 minutes and should preferably be 4—10 minutes, all depending on pressure and temperature conditions chosen.

Taking into account that the following sulphation process is preferably undertaken in a non aqueous environment, it is desirable to recover the polysaccharide in a waterless form from the extract. This is done either by direct freeze-drying of the extract or by addition of a precipitant for the polysaccharide, e.g. acetone or an alcohol, preferably ethanol, in a volume which is sufficient for the complete precipitation of the polysaccharide. Usually, an equal volume of alcohol is added to the extract. The precipitate formed is collected and dried, then the dry precipitate is dissolved in a non aqueous solvent e.g. formamide, whereafter the sulphation process can be carried out.

Preferably, the extracted polysaccharide material is subjected to a hydrolysis step for partial depolymerization i.e. to reduce the molecular weight below 70.000, preferably in the range of 2.500 to 40.000 and more preferably in the range of 5.000 t0 30.000. This hydrolysis step is suitably carried out prior to the sulphation process. The hydrolysis is performed with acid, e.g. in a 1 molar hydrochloric acid solution, at elevated temperature, e.g. between 60 and 100°C, for 5 minutes to several hours, e.g. 15 minutes at 90°C. After the hydrolysis the solution is filtered, a precipitant is added, e.g. an equal volume of an alcohol, preferably ethanol, and the precipitate is collected, dried and then sulphated.

As mentioned above, the sulphation process described in the invention is preferably carried out by treating the polysaccharide with chlorosulphonic acid in the presence of pyridine. However, the invention is not limited to that process, and every process leading to sulphation lies within the scope of the invention. Such a sulphation process is known, and the US patent 2 599 172 may, for instance, be referred to. It describes the sulphation of the mucopolysaccharide hyaluronic acid with the aid of chlorosulphonic acid or sulphuric acid. Chlorosulphonic acid is preferably chosen as sulphation agent in connection with the invention. The reaction is carried out in the presence of a nitrogen base, e.g. pyridine, which takes up produced hydrochloric acid gas, whereby the reaction balance is changed in the direction required. On adding chlorosulphonic acid to the reaction solution an exothermic reaction is produced, whereby the temperature of the reaction spontaneously rises to approximately 60—90°C. The warm reaction solution is then allowed to cool down to room temperature, which takes about 2 hours, thereafter the reaction product is precipitated by the addition of a precipitant, e.g. acetone or alcohol. Ethanol is preferably added as precipitant. The precipitate obtained is then washed, e.g. with ethanol, in order to eliminate pyridine. In order to cleanse the product still further, and also in order to convert it into a pharmaceutically acceptable salt, which is often desirable, the precipitate is dissolved in water, then precipitated again with a precipitant as indicated hereinbefore. Thereafter, the precipitate is processed for conversion to a required salt e.g. with sodium acetate for conversion to sodium salt. Finally, the product is purified by filtration through activated charcoal and liberated from low molecular salts by a generally known method, e.g. dialysis.

Of the various pharmaceutically acceptable salts, the sodium salt is particularly suitable for use in vivo. However other salts can also be used, e.g. potassium salt, calcium salt etc.

For medicinal purposes the new anticoagulant can be administered enterally (i.e. orally, particularly sublingually), parenterally (i.e. subcutaneously, intramuscularly, particularly intravenously) or topically. Normally, it is applied in the usual dosage forms for anticoagulants such as heparin, for example intravenously in aqueous solutions, e.g. physiological salt solutions, glucose solutions or mucopolysaccharide solutions. Such solutions are usually ampouled, the vials being filled under aseptic conditions. The sterile solutions may contain preservatives such as benzylalcohol, chlorobutanol and those of the phenolic type, e.g. cresol. Also, the new anticoagulant may be stored in vials in the lyophilized state (obtained by freeze drying), said form being ready for making injectable solutions just prior to administration.

For oral or sublingual application the new anticoagulant, preferably in the pharmaceutically acceptable salt-form, is administered in admixture with suitable excipients in the form of tablets, pills, dragees, capsules and the like.

For topical application the new anticoagulant is administered in the form of creams, ointments or lotions, usually also containing a penetration agent such as dimethyl sulphoxide, for improving or accelerating the penetration through the skin.

The new anticoagulant can also be administered in combination with other biologically active substances, such as vitamins, ephedrin, dihydroergotaminmethanesulphonate.

In order to further illustrate the invention, the following experimental examples are described, which should not be considered as a limitation of the invention.

### Example 1

150 g fresh sea mussel (Mytilus edulis) were boiled under a pressure of 4 atm (405.3 kPa) during 4 minutes in 100 ml 0.9% sodium chloride solution, then cooled down to room temperature. The decoction obtained amounted to 102 ml and was filtered through filter paper. By adding an equal volume of 99.5% ethanol to the decoction after filtration, a thick, grey-white precipitate was obtained. The precipitate was centrifuged at 5000 r/m at +4°C during 20 minutes, whereupon the upper layer was removed and the precipitate was collected. The grey-white precipitate was then dissolved in 50 ml distilled water and all unsolved material removed by simple paper filtration. Thereafter the solution was freeze-dried whereupon 5.61 g dry grey-white powder was collected. This powder was dissolved in 50 ml formamide during 12 hours at +4°C whereafter 50 ml pyridine was added to the solution under agitation. Then 10 ml chlorosulphonic acid was added, whereupon the reaction solution turned deeply dark-coloured while the temperature rose spontaneously to 90°C. Following the rise in temperature, the reaction solution was cooled to room temperature, whereupon an equal volume of 99.5% ethanol was added and the precipitate formed was collected after centrifugation. The precipitate was washed thrice in absolute ethanol and then dissolved in 100 ml distilled water. Thereupon 1.35 g N-cetyl-N', N',N'-trimethyl-ammonium-bromide was added under agitation. The solution was acidified with sulphuric acid to pH 2. A thick, white precipitate was formed which was collected after centrifugation and dissolved in 100 ml butanol. The butanol phase was extracted with 20 ml of a 2% sodium acetate solution, whereupon the lower water phase was collected and the salt removed through dialysis with distilled water on a molecule sieve, brand name Diaflo (from Diaflo Inc., USA) with filter DM 5. The filter allowed molecules with a molecular weight below 5000 to pass through. Following concentration and freeze-drying, 0.57 g product was obtained and this was dissolved in water and then filtered through activated charcoal in order to eliminate colloidal particles. After renewed freeze-drying, 135.7 mg of an extremely soluble substance with considerably anticoagulant action remained.

### Example 2

The substance obtained in Example 1 was closely investigated regarding its anticoagulant action. 0.19—0.15 mg of the substance per ml freshly collected human whole blood gave no blood coagulation under more than 24 hours at room temperature. Partial thromboplastin time (APTT from General Diagnostics USA) showed 8 times weaker activity compared with commercial heparin (140 IU/mg; Vitrum AB, Sweden). The activity toward factor X (Pharmacia test) showed no activity at all. In a system containing 0.4% fibrinogen without other plasma factors present, the new substance was added together with pure thrombin (Topostatin [R] from Hoffman-la Roche, Switzerland) in various quantities. In these titration experiments it was found that 0.1 mg/ml of the new substance totally inhibited the conversion of fibrinogen into fibrin by thrombin. In similar experiments with heparin no effect whatsoever was noted. This shows that the new substance is a specific thrombin inhibitor which does not require the presence of plasma cofactor in order to exert its inhibition. The titration experiments indicate that 1.5 NIH-units thrombin (NIH = National Institute of Health) are neutralized by 0.1 mg of the new thrombin inhibitor. An adult with approximately 3 liters of plasma should thus need about 300 mg of the new inhibitor, should a 100% extinction of the thrombin activity be required. The content of prothrombin in human plasma is about 90 mg/1, i.e. 270 mg/3 l plasma. In view of these data, it is likely that the new inhibitor forms a stable 1:1 complex with thrombin.

## Example 3

The thrombin inhibitor described in Example 1 was investigated in a series of adult rats weighing an average of 200 g each. Each rat received 5 mg of the thrombin inhibitor intravenously and clotting time, the so-called thrombin time, was recorded every hour. Under 5 hours thrombin time was considerably prolonged. A series of similar investigation was carried out with intravenous injection of 5 mg commercial heparin (Vitrum AB) in another group of rats. An identical increase of the prothrombin time was noted but with the difference that the commercial heparin had one hour longer duration.

## Example 4

A comparative investigation of the thrombin inhibitor of Example 1 with commercial heparin with respect to its character as a sulphated polysaccharide showed no substantial differences between the two substances. Just as for commercial heparin, the new thrombin inhibitor was correspondingly precipitated by barium chloride, lead acetate and protamine and showed even considerable metachromasia with toluidine blue (blue-violet). The thrombin inhibitor was also lightly colourable with azure A. Analysis revealed that the inhibitor described in Example 1 had a sulphate content of 12—16%. Electrophoresis in veronal buffer pH 8.6 on agarose revealed a clearly distinct band with a slightly higher mobility than heparin (Vitrum AB). Just as for commercial heparin, the thrombin inhibitor was colourable with neutral red and methylene blue and exhibited positive naphthol and orcinol reaction respectively.

## Example 5

The thrombin inhibitor was prepared as described in Example 1 but instead of Mytilus edulis, oysters (Limfjorden, Denmark) were used as initial material. After boiling 1 kg oysters and processing the decoction in a similar way as described in Example 1, 1.5 g of the inhibitor substance were collected with exactly the same biological and physical properties as for the previous examples.

## Example 6

The procedure described in Example 1 was followed but instead of Mytilus edulis 150 g common sea mussel from South Korea were used as initial material. 130 mg of the inhibitor substance were obtained with exactly the same biological and physical properties as for the thrombin inhibitor described in Example 1.

## Example 7

The procedure described in Example 1 was followed but instead of Mytilus edulis 200 g mussel of the Cyprina islandica species were used. 150 mg of the inhibitor substance were obtained with exactly the same biological and physical properties as for the thrombin inhibitor described in Example 1.

## Example 8

1.2 kg mussel flesh were boiled in 200 ml 0.1 I M NaOH under 12 minutes at atmospheric pressure. The decoction was filtered then allowed to cool down to room temperature. Thereafter an equal volume of 99.5% ethanol was added, whereupon a grey-white precipitate was formed. The precipitate was centrifuged with 500 r/m at +4°C during 20 minutes, whereupon the upper layer was removed and the precipitate collected. Freeze-drying of the precipitate yielded 4.6 g of a dry, grey-white powder. This powder was sulphated according to the method described in Example 1 with chlorosulphonic acid in the presence of pyridine. Further processing gave 102.2 mg of the anticoagulant according to the invention.

## Example 9

An extract, which was collected through boiling mussel as described in Example 1, was freeze-dried. 81 mg of the freeze-dried substance was dissolved in 1.6 ml distilled water and 81 mg $Na_2CO_3$ was added under agitation.

A complex of pyridine and sulphur trioxide was prepared by adding 10 ml pyridine by drip in a glazed earthenware pot together with 2 ml chlorosulphonic acid. The complex precipitated at the bottom of the glazed earthenware pot as a precipitate of cristalloid type.

90 mg of the complex was added to the above water solution and the whole solution was allowed to react under 2 hours at +20°C. Thereafter, 1,4 ml 4 M NaCl was added and the solution was relieved from low molecular salt by dialysis. Freeze-drying of the dialyzed solution yielded 46.2 mg of the anticoagulant agent according to the invention.

On investigation, it was found that 1.25 mg of the collected product thus formed inhibited clotting of 5 ml of human venous blood in vitro during more than 7 days at room temperature. On the other hand, no prolongation of the clotting time in vitro was obtained with 0.6 mg of the product mixed with 5 ml blood. The product was investigated in a system without cofactor, as described in Example 2, whereby it was established that the anticoagulant effect in vitro was due to a selective inhibition of thrombin.

## Example 10

1500 g fresh mussels from Tjärnö, Sweden, with shells were boiled in 2000 ml water in a high-pressure cooker (2 atm) (202.65 kPa) for 30 minutes and then cooled down to room temperature. Filtration gave 2210 ml decoction water. By adding an equal volume of 99.5% ethanol a thick, grey-white precipitate was obtained. The precipitate was collected by centrifugation at 7000 rpm at 4°C for 10 minutes. The precipitate was dissolved in 500 ml water and 40 ml conc. HCl-solution was added. The acid solution is kept on a water bath at 90°C for 15 minutes and then cooled down to 4°C. After filtration an equal volume of 99.5% ethanol is added and the precipitate obtained is collected by centrifugation at 7000 rpm at 4°C for 20 minutes.

Sulphation of the precipitate and further processing according to a similar procedure as described in Example 1 gave 5.62 g product of the invention with similar biological properties to the thrombin inhibitor of Example 1.

### Example 11

130 g preserved octopus and 10 ml liquid were admixed with 500 ml distilled water and then boiled for 30 minutes at a pressure of 2 atm (202.65 kPa). The boiling water was tested with regard to toluidine-metachromasy, addition of barium chloride, lead acetate and protamine. All tests were negative, which showed that no sulphate was present. An equally large volume of absolute (99.5%) ethanol was then added to the boiling water, giving a precipitate to which concentrated HCl was added until the solution was 1 M with regard to HCl, and the solution was then heated for 15 minutes at 90°C. One and a half times as large a volume of absolute ethanol was then added and the resulting precipitate was collected and freeze-dried.

A yield of 95.4 mg of a pure white fluffy powder was obtained. The dried powder was admixed with 19 ml formamide and 19 ml pyridine, after which 3.8 ml chlorosulphonic acid was added dropwise under agitation. The temperature spontaneously rose to 90°C during 5 minutes, and the preparation was then allowed to cool to room temperature. pH was adjusted from 4.1 to 7.0 by means of 6 M NaOH.

The product thus sulphated was precipitated by addition of an equally large volume of absolute ethanol. After desalinisation by ultrafiltration (cut off at molecular weight = 10.000) the precipitate was freeze-dried, the final yield being 126 mg.

When testing the product with regard to prolongation of the thrombin time in a plasma system (containing the cofactor antithrombin III), one found that the product was twenty times weaker than heparin. When testing the product in a system free of cofactors one obtained a considerable prolongation of the thrombin time. Heparin on the other hand gave no prolongation of the thrombin time in this system.

### Example 12

125 g preserved snails and 95 ml liquid were boiled and treated under the same conditions as in Example 11, 44 mg unsulphated powder being obtained after freeze drying. This powder was then sulphated under the same conditions as in Example 11 with chlorosulphonic acid in the presence of formamide and pyridine.

69.3 mg final product were obtained after dialysis and freeze-drying.

The anticoagulation ability of the final product was examined by measurement of the thrombin time prolongation respectively in human plasma (contains a cofactor) and in a fibrinogen system (lacks a cofactor).

It was found that the product had an anticoagulating effect also in the absence of a cofactor.

Thus the product had a strong anticoagulation effect in the cofactor-free fibrinogen system, heparin being totally devoid of any effect. In the plasma system, which contained a cofactor, the product showed an anticoagulation ability twenty times lower than that of heparin.

### Example 13

In laboratory animals, experimental thrombosis can be induced by bringing blood into contact with foreign thrombogenic surfaces. A simple, reproducible method for the induction of experimental thrombosis in rats is the insertion of a silk thread in an extracorporeal shunt between the carotid artery and the jugular vein. (T. Umetsu & K. Sanai; International Journal on Haemostatis and Thrombosis Research *39*, (1978) 74—83).

The weight of the formed thrombi is a measure for the severity of the thrombosis. Heparins and heparin-like materials inhibit the formation of these thrombi.

*Antithrombosis-test (3 rats)*

The weight of the thrombi was reduced by 75% and 88% after a single i.v. bolus injection with 6.4 mg/kg and 12.8 mg/kg respectively of the product of Example 10 as compared to placebo treatment.

The product of Example 10 had sulphate content of about 38% and a mean molecular weight of about 37.000.

### Example 14

15.4 kg fresh mussels from Zeeland, the Netherlands, with shells and 20.5 l water were boiled in an autoclave at 120°C for 30 minutes. Simple filtration at room temperature gave 21 l decoction water. By adding an equal volume of .99.5% ethanol at 4°C a thick, grey-white precipitate was obtained. The wet precipitate (420 g) was divided in two portions. The first portion was hydrolyzed in 1 M hydrochloric acid solution for 15 minutes at 90°C, the second portion in 1.2 M hydrochloric acid solution for 30 minutes at 90°C. The hydrolyzed products were precipitated with 99.5% ethanol (1:1) and the precipitates collected by centrifugation. The first precipitate was freeze-dried, the second precipitate was dried in vacuum at room temperature. Sulphation of each precipitate (about 12 g) was carried out with chlorosulphonic acid (400 ml) in a mixture of 2 l

formamide and 2 l pyridine for 5 minutes. After cooling the pH is adjusted to 7.0 with 6 M NaOH-solution. Precipitation with 99.5% ethanol (1:1) at 4°C and centrifugation gave 23.4 g product I and 19.6 g product II. Both products had a sulphate content of about 50%. Mean molecular weight of product I was about 40.000, of product II about 16.000.

Testing of the products as in Example 2 showed the specific thrombin inhibiting action without cofactor.

In the anti-thrombosis test of Example 13 the following data were found:

Product I: The weight of the thrombi was reduced by 80% and 91% after a single i.v. bolus injection with 8 mg/kg and 16 mg/kg of product, respectively. For product II the percentages were 74% and 91%, respectively.

**Claims**

1. Novel anticoagulant having selective thrombin inhibiting action in the absence of cofactor characterized by the fact that it is a sulphated polysaccharide which is prepared by (a) extracting in aqueous solution a polysaccharide material from mollusca, (b) optionally subjecting said polysaccharide material to acid hydrolysis so as to reduce the molecular weight below 70000, (c) sulphating said polysaccharide material or said hydrolysed polysaccharide material and (d) optionally converting the sulphated material into a pharmaceutically acceptable salt thereof.

2. The anticoagulant of claim 1, in the preparation of which mussels are used as material to be extracted.

3. The anticoagulant of claim 1, in the preparation of which common sea mussels (Mytilus) are used as material to be extracted.

4. The anticoagulant of claims 1 to 3 in the preparation of which hydrolysis is performed so as to reduce the molecular weight to be in the range of 2500 to 40000.

5. The anticoagulant of claim 4 in the preparation of which the molecular weight is reduced so as to be in the range of 5000 to 30000.

6. The anticoagulant of claims 1 to 5 in the preparation of which the sulphated material is converted into the sodium salt thereof.

7. Process for preparing the anticoagulant of claims 1 to 6 by (a) extracting in aqueous solution flesh from mollusca for obtaining polysaccharide material included therein, (b) optionally subjecting said polysaccharide material to acid hydrolysis so as to reduce the molecular weight below 70000, (c) sulphating said polysaccharide material or said hydrolysed polysaccharide material according to the methods known in the art and (d) optionally converting the sulphated material into a pharmaceutically acceptable salt thereof.

8. Process according to claim 7, wherein the extraction is performed in aqueous solution at a pressure in the range of 2 to 15 atm, (202.65 to 1519.875 kPa), at a temperature of at least 100°C and for a period of 2 to 15 minutes.

9. Process according to claim 8, wherein the pressure is in the range of 4 to 6 atm (405.3 to 607.95 kPa).

10. Process according to claim 8 or 9, wherein the temperature is the temperature corresponding to saturated steam pressure at chosen pressure.

11. Process according to claims 7—10 wherein the acid hydrolysis is performed so as to reduce the molecular weight to be in the range of 2500 to 40000.

12. Process according to claim 11, wherein the molecular weight is reduced to be in the range of 5000 to 30000.

13. Process according to claims 7—12, wherein the sulphated product is converted into the sodium salt thereof.

14. A pharmaceutical preparation having selective thrombin inhibiting action in the absence of cofactor comprising the anticoagulant of claims 1—6, the usual ingredients for pharmaceutical preparations and optionally other biologically active substances.

**Revendications**

1. Nouvel anticoagulant ayant une action sélective d'inhibition de la thrombine en l'absence de cofacteur, caractérisé par le fait qu'il est un polysaccharide sulfaté que l'on a préparé en (a) extrayant dans une solution aqueuse une matière polysaccharidique de mollusques, (b) soumettant éventuellement ·ladite matière polysaccharidique à une hydrolyse acide pour abaisser le poids moléculaire en dessous de 70 000, (c) transformant en ester sulfurique ladite matière polysaccharidique ou ladite matière polysaccharidique hydrolysée et (d) transformant éventuellement la matière sulfatée en un sel acceptable en pharmacie.

2. Anticoagulant selon la revendication 1, dans la préparation duquel on utilise des moules comme matière à extraire.

3. Anticoagulant selon la revendication 1, dans la préparation duquel on utilise des moules de mer communes (Mytilus) comme matière à extraire.

4. Anticoagulant selon l'une des revendications 1 à 3, dans la préparation duquel on effectue une hydrolyse pour abaisser le poids moléculaire dans la gamme de 2 500 à 40 000.

5. Anticoagulant selon la revendication 4, dans la preparation duquel on abaisse le poids moléculaire pour qu'il soit compris dans la gamme de 5 000 à 30 000.

6. Anticoagulant selon l'une des revendications 1 à 5, dans la préparation duquel on transforme en le sel de sodium la matière sulfatée.

7. Procédé pour préparer l'anticoagulant selon l'une des revendications 1 à 6 en (a) extrayant en solution aqueuse la chair de mollusques pour obtenir la matière polysaccharidique qui y est contenue, (b) soumettant éventuellement ladite matière polysaccharidique à une hydrolyse acide pour abaisser le poids moléculaire en dessous de 70 000, (c) transformant en ester sulfurique ladite matière polysaccharidique ou ladite matière polysaccharidique hydrolysée selon des procédés connus dans l'art et (d) transformant éventuellement la matière transformée en ester sulfurique en un sel acceptable en pharmacie.

8. Procédé selon la revendication 7, dans lequel on effectue l'extraction en solution aqueuse à une pression comprise dans la gamme de 202,65 à 1 519,875 kPa à une température d'au moins 100°C pendant une durée de 2 à 15 minutes.

9. Procédé selon la revendication 8, dans lequel la pression est comprise dans la gamme de 405,3 à 607,95 kPa.

10. Procédé selon l'une des revendications 8 ou 9, dans lequel la température est la température correspondant à la pression de vapeur saturée à la pression choisie.

11. Procédé selon l'une des revendications 7 à 10, dans lequel on effectue l'hydrolyse acide de façon à abaisser le poids moléculaire dans la gamme de 2 500 à 40 000.

12. Procédé selon la revendication 11, dans lequel on abaisse le poids moléculaire dans la gamme de 5 000 à 30 000.

13. Procédé selon l'une des revendications 7 à 12, dans lequel on transforme en le sel de sodium le produit transformé en ester sulfurique.

14. Préparation pharmaceutique ayant une action sélective d'inhibition de la thrombine en l'absence de cofacteur, comprenant un anticoagulant selon l'une des revendications 1 à 6, les ingrédients habituels des préparations pharmaceutiques et éventuellement d'autres substances à activité biologique.

**Patentansprüche**

1. Neue Anticoagulans, welches selektive thrombinhemmende Wirkung in Abwesenheit von Cofaktor aufweist, dadurch gekennzeichnet, dass es ein sulfatiertes Polysaccharid ist, welches hergestellt wird durch (a) Extrahieren in wässeriger Lösung eines Polysaccharidmaterials aus Weichtieren, (b) gegebenenfalls saure Hydrolyse des Polysaccharidmaterials, um das Molekulargewicht unter 70'000 herabzusetzen, (c), Sulfatieren des Polysaccharidmaterials oder des hydrolysierten Polysaccharidmaterials, und (d) gegebenenfalls Umwandlung des sulfatierten Materials in ein pharmazeutisch annehmbares Salz davon.

2. Anticoagulans nach Anspruch 1, zu dessen Herstellung Muscheln als das zu extrahierende Material verwendet werden.

3. Anticoagulans nach Anspruch 1, zu dessen Herstellung Miesmuscheln (Mytilus) als das zu extrahierende Material verwendet werden.

4. Anticoagulans nach den Ansprüchen 1 bis 3, zu dessen Herstellung die Hydrolyse derart durchgeführt wird, dass das Molekulargewicht auf dem Bereich von 2500 bis 40'000 herabgesetzt wird.

5. Anticoagulans nach Anspruch 4, bei dessen Herstellung das Molekulargewicht derart herabgesetzt wird, dass es im Bereich von 5000 bis 30'000 liegt.

6. Anticoagulans nach den Ansprüchen 1 bis 5, bei deseen Herstellung das sulfatierte Material in das Natriumsalz davon umgewandelt wird.

7. Verfahren zur Herstellung des Anticoagulans nach den Ansprüchen 1 bis 6, durch (a) Extrahieren in wässeriger Lösung von Fleisch von Weichtieren, um das darin enthaltene Polysaccharidmaterial zu erhalten, (b) gegebenenfalls saure Hydrolyse dieses Polysaccharidmaterials, um das Molekulargewicht auf unter 70'000 herabzusetzen, (c) Sulfatieren des Polysaccharidmaterials oder des hydrolysierten Polysaccharidmaterials nach bekannten Methoden und (d) gegebenenfalls Umwandlung des sulfatierten Materials in ein pharmazeutisch annehmbares Salz davon.

8. Verfahren nach Anspruch 7, dadurch gekennzeichnet, dass die Extraktion in wässeriger Lösung einem Druck im Bereich von 2 bis 15 Atmosphären (202,65 bis 1 519,875 kPa) bei einer Temperatur von mindestens 100°C und während einer Zeit von 2 bis 15 Minuten durchgeführt wird.

9. Verfahren nach Anspruch 8, dadurch gekennzeichnet, dass der Druck im Bereich von 4 bis 6 Atmosphären (405,9 bis 607,95 kPa) liegt.

10. Verfahren nach Anspruch 8 oder 9, dadurch gekennzeichnet, dass die Temperatur die im gesättigten Dampfdruck bei gewähltem Druck entsprechende Temperatur ist.

11. Verfahren nach den Ansprüchen 7 bis 10, dadurch gekennzeichnet, dass die saure Hydrolyse derart durchgeführt wird, dass das Molekulargewicht auf einem Bereich von 2500 bis 40'000 herabgesetzt wird.

12. Verfahren nach Anspruch 11, dadurch gekennzeichnet, dass das Molekulargewicht derart reduziert wird, dass es im Bereich von 5000 bis 30'000 liegt.

13. Verfahren nach den Ansprüchen 7 bis 12, dadurch gekennzeichnet, dass das sulfatierte Produkt in das Natriumsalz davon umgewandelt wird.

14. Pharmazeutisches Präparat mit selektiver thrombinhemmender Wirkung in Abwesenheit eines Cofaktors, enthaltend das Anticoagulans der Ansprüche 1 bis 6, die üblichen Ingrendienzien für pharmazeutische Präparate und gegebenenfalls andere biologische aktive Substanzen.